# EUROPEAN PATENT APPLICATION

(11) **EP 2 072 514 A1**
(43) Date of publication of application: **24.06.2009**
(21) Application number: 07150077.1
(22) Date of filing: 17.12.2007
(51) Int. Cl.: C07D 413/10, A61K 31/422, A61P 31/04

(54) **1(2)H-tetrazol-5-yl-phenyl-oxazolidinones as antibacterial agents**

(71) Applicant: Ferrer Internacional, S.A., 08028 Barcelona (ES)
(72) Inventor: Cano, Montserrat, 08691, Monistrol (Barcelona) (ES); Palomer, Albert, 08014, Barcelona (ES); Guglietta, Antonio, 08750, Molins de Rei (Barcelona) (ES)
(74) Representative: Barlocci, Anna

(57) **Abstract**

The present invention provides a class of phenyl-oxazolidinones of formula (I): 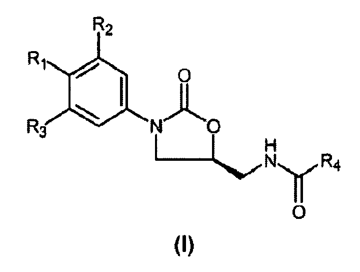 or a pharmaceutically acceptable salt thereof, wherein:
R₁ is a radical of formula (i) or formula (ii): 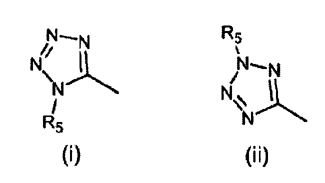 methods of preparing such compounds, pharmaceutical compositions comprising one or more of such compounds, and the use thereof in the manufacture of a medicament which is used in the treatment of bacterial infections.

## Description

### TECHNICAL FIELD

This invention is directed to antimicrobial oxazolidinone compounds which are active against Gram-positive and some Gram-negative bacteria and have a weak monoamine oxidase (MAO) inhibitory activity.

### BACKGROUND ART

Oxazolidinones were originally developed as MAO inhibitors for treatment of depression and Parkinson's disease. MAO is one of the primary enzymes responsible for the catabolism of catecholamines. In humans, MAO occurs in two isoforms, MAO-A and MAO-B. MAO-A preferentially deaminates serotonin (5-HT) and norepinephrine; MAO-B preferentially deaminates phenylethylamine, benzylamine, and, in man, dopamine. Normally MAO-A inhibitors, such as moclobemide or tranylcypromine, have been used as antidepressant agents while MAO-B inhibitors, such as selegiline, have been used preferably in the therapy of Parkinson's disease. US Patent 3655687 discloses 5-hydroxymethyl-3-substituted-2-oxazolidinone derivatives with significant antidepressant activity. A compound disclosed in this patent, toloxatone, 5-(hydroxymethyl)-3-(3-methylphenyl)-2-oxazolidinone, is of particular relevance.

Oxazolidinones are also Gram-positive antimicrobial agents. Oxazolidinones bind to the 50S subunit of the prokaryotic ribosome, preventing formation of the initiation complex for protein synthesis. This is a novel mode of action. Other protein synthesis inhibitors either block polypeptide extension or cause misreading of mRNA. Linezolid (N-[[(5S)-3-[3-fluoro-4-(4-morpholinyl)phenyl]-2-oxo-5-oxazolidinyl]methyl]acetamide, US5688792) was the first approved antimicrobial oxazolidinone for clinical use in the United States and elsewhere.

Linezolid minimal inhibitory concentrations (MICs) vary slightly with the test mode, laboratory, and significance attributed to thin hazes of bacterial survival, but all workers find that the susceptibility distributions are narrow and unimodal with MIC values between 0.5 and 4 µg/mL for *streptococci, enterococci* and *staphylococci.* Full activity is retained against Gram-positive *cocci* resistant to other antibiotics, including methicillin-resistant *staphylococci* and vancomycin-resistant *enterococci.* MICs are 2-8 µg/mL for *Moxarella, Pasteurella* and *Bacteroides* spp. but other Gram-negative bacteria are resistant as a result of endogenous efflux activity as well as the intake presented by Gram-negative bacteria outer membrane cell. Linezolid is indicated for the treatment of adult patients with the following infections:
Vancomycin-resistant *Enterococcus faecium* infections, including concurrent bacteremia; nosocomial pneumonia; complicated skin and skin structure infections; community-acquired pneumonia, including concurrent bacteremia; diabetic foot infections; and uncomplicated skin and skin structure infections.

It is well-known for the skilled man in the art that when a drug (e.g. an oxazolidinone with antibacterial activity) is added to an already therapeutic regimen, the risk of interaction drug-drug is substantially increased. The main consequence of the interaction between two drugs is that the pharmacological effect is modified by the presence of the second drug. The result of this interaction can be harmful for the patient since said interaction can cause toxicity or a decrease in the efficiency of one the drugs ingested by the patient. Because of this, particular attention has been paid to the question of possible adverse interactions with drugs known to be metabolized by monoamine oxidase in patients who are being treated with oxazolidinones. For instance, it has been observed that linezolid (an oxazolidione with antibacterial activity) causes an enhanced response in patients who are taking certain adrenergic agents, including phenylpropanolamine and pseudoephedrine, and it is specifically noted in the leafleft that the doses of these drugs should be reduced in patients receiving linezolid. The package insert for linezolid warns against combining it with tyramine-rich foods and about being aware of a potential interaction with adrenergic and serotonergic agents and also that the side effects of linezolid may be increased by certain drugs including, among others, monoamine oxidase (MAO) inhibitors (such as selegiline, isocarboxazid, pargyline, phenelzine, and tranylcypromine), being recommended that these combinations must be avoided.

It has been proposed the use of oxazolidinones having a weak MAO activity in order to minimize the secondary effects due to the drug-drug interactions, such as disclosed in American patents US6083967, US6265178, and US6605609 and in PCT applications W02005113520, W02006010756, W02006016220, W02006016221, W02006106426, W02006106427, W02007000432, and W02007000644.

On the other hand, some tetrazolyl-phenyl-oxazolidinones have been disclosed in literature. The compounds of the present invention, although similar to prior art products, exhibit a characteristic Carbon-Carbon linked tetrazolyl-phenyl moiety, i.e. a 1 H-tetrazol-5-yl- or a 2H-tetrazol-5-yl- fragment.

The American patent US5910504 discloses oxazolidinones of formula (A) 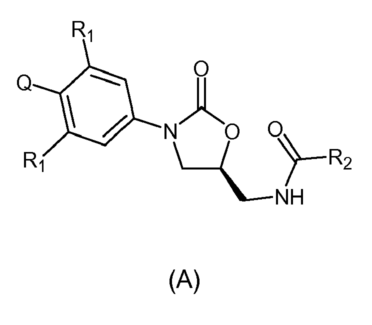 wherein Q is a hetero-aromatic 5 member ring linked to the benzene ring by a Nitrogen-Carbon bond, and R₁-R₂ have different values. The only two tetrazole compounds of formulas 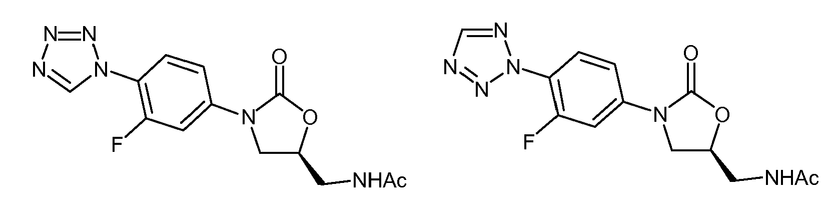 have been mentioned in the specification, being designed as (uu) and (vv) respectively. No preparative details, physico-chemical constants nor microbiological or MAO activities are given for them.

The PCT application W02005042523 discloses oxazolidinones of formula (B) 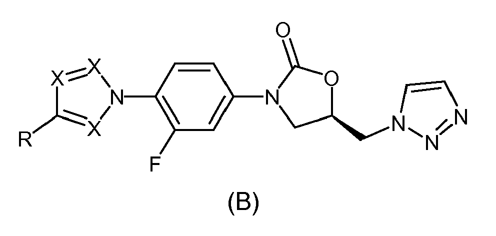 wherein R and X have different values, which are antibacterial agents. No MAO activities are given for any compound. The only tetrazole compound (R: H, X: N) in the invention is disclosed in Example 10.

Genin M. J. et al. (J. Med.Chem. 2000, 43, 953-970) discussed the substituent effects on the antibacterial activity of Nitrogen-Carbon linked (Azolylphenyl)oxaxolidinones with expanded activity against the fastidious Gram-negative organisms *Haemophilus influenzae* and *Moraxella catarrhalis.* Pyrrole, pyrazole, imidazole, triazole and tetrazole moieties were used to replace the morpholine ring of linezolid. In the tetrazole series the 5-cyano-2*H-*tetrazole derivative of formula (C) 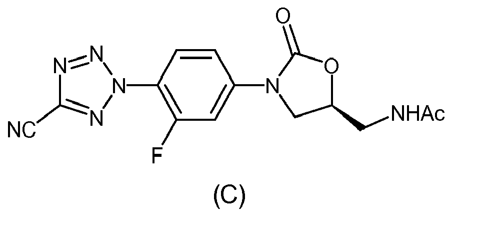 was the most relevant one, showing good Gram-positive activity, but it showed no appreciable benefit over linezolid. No MAO activities are given for any compound.

Despite the teaching of prior art, the research of new antibacterial agents showing a weak inhibitory MAO activity is still an active field.

### SUMMARY OF THE INVENTION

In its many embodiments, the present invention provides a novel class of phenyl-oxazolidinones as antibacterial agents, methods of preparing such compounds, pharmaceutical compositions comprising one or more of such compounds, and the use thereof in the manufacture of a medicament which is used in the treatment of bacterial infections.

In one aspect, the present invention refers to a compound of formula (I), 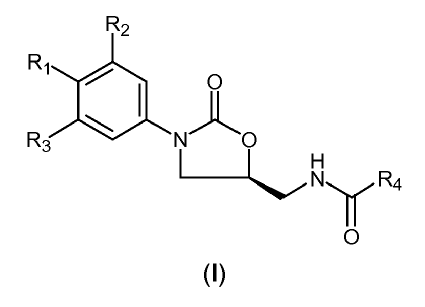 or a pharmaceutically acceptable salt thereof, wherein:
R₁ is a radical of formula (i) or formula (ii): 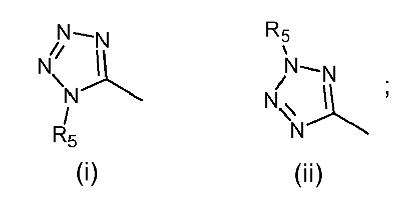
R₂ and R₃ are radicals identical or different and are independently selected from hydrogen and fluorine;
R₄ is a linear or branched alkyl (C₁-C₆) radical optionally substituted by 1 to 3 fluorine atoms;
R₅ is a radical selected from the group consisting of hydrogen; linear or branched alkyl (C₁-C₆) optionally substituted by OH, OR₆, NO₂, CF₃, CN, COOH, COOR₇, COR₈, CONH₂, CONHR₉, and CONR₁₀R₁₁; and linear or branched alkynyl (C₁-C₆);
R₆ is a linear or branched alkyl (C₁-C₆) radical;
R₇ is a linear or branched alkyl (C₁-C₆) radical;
R₈ is a linear or branched alkyl (C₁-C₆) radical;
R₉ is a linear or branched alkyl (C₁-C₆) radical; and
R₁₀ and R₁₁ are linear or branched alkyl (C₁-C₆) radicals which are identical or different or taken together can form with the nitrogen atom to which they are attached a pyrrolidine or piperidine ring.

A second aspect of the present invention is the provision of several processes for the preparation of a compound of formula (I) comprising the steps mentioned in the detailed description of the invention.

A third aspect of the present invention is the provision of a pharmaceutical composition comprising at least one compound of formula (I), together with appropriate amounts of pharmaceutical excipients or carriers.

Finally, a four aspect of the present invention is to provide the use of a compound of formula (I) for the manufacture of a medicament for the treatment of bacterial infections in a human or animal.

### DETAILED DESCRIP!ION OF THE INVENTION

In a first aspect, the present invention discloses 1 (2)H-tetrazol-5-yl-phenyl-oxazolidinones which are represented by the structural formula (I), or a pharmaceutically acceptable salt thereof, wherein the various moieties are as described above.

In a preferred embodiment, at least one of R₂ and R₃ is fluorine.

In another preferred embodiment, R₄ is a linear or branched alkyl (C₁-C₆) radical.

In another preferred embodiment, R₅ is hydrogen; a linear or branched alkyl (C₁-C₆) radical optionally substituted by OH, OR₆, CN, COOH, COOR₇, and COR₈; or a linear or branched alkynyl (C₁-C₆) radical.

In a more preferred embodiment, compound of formula (I) is one of the following list:
*N*-[[(5S)-3-[3-fluoro-4-(1*H*-tetrazol-5-yl)-phenyl]-2-oxo-5-oxazol id inyl] methyl]acetam ide;
*N*-[[(5S)-3-[3-fluoro-4-(1-methyl-1*H*-tetrazol-5-yl)-phenyl]-2-oxo-5-oxazol id inyl] methyl]acetam ide;
*N*-[[(5S)-3-[3-fluoro-4-(2-methyl-2*H*-tetrazol-5-yl)-phenyl]-2-oxo-5-oxazol id inyl] methyl]acetam ide;
*N*-[[(5S)-3-[3-fluoro-4-(2-cyanomethyl-2*H*-tetrazol-5-yl)-phenyl]-2-oxo-5-oxazol id inyl] methyl]acetam ide;
*N*-[[(5S)-3-[3-fluoro-4-(1-cyanomethyl-1*H*-tetrazol-5-yl)-phenyl]-2-oxo-5-oxazolidinyl]methyl]acetamide
*N*-[[(5S)-3-[3-fluoro-4-(2-acetoacetyl-2*H*-tetrazol-5-yl)-phenyl]-2-oxo-5-oxazol id inyl] methyl]acetam ide;
*N*-[[(5S)-3-[3-fluoro-4-(2-hydroxyethyl-2*H*-tetrazol-5-yl)-phenyl]-2-oxo-5-oxazol id inyl] methyl]acetam ide;
*N*-[[(5S)-3-[3-fluoro-4-(1-hydroxyethyl-1*H*-tetrazol-5-yl)-phenyl]-2-oxo-5-oxazol id inyl] methyl]acetam ide;
*N*-[[(5S)-3-[3-fluoro-4-(2-methoxyethyl-2*H*-tetrazol-5-yl)-phenyl]-2-oxo-5-oxazol id inyl] methyl]acetam ide;
*N*-[[(5S)-3-[3-fluoro-4-(1-methoxyethyl-1*H*-tetrazol-5-yl)-phenyl]-2-oxo-5-oxazol id inyl] methyl]acetam ide;
*N*-[[(5S)-3-[3-fluoro-4-(2-methoxycarbonylmethyl-2*H*-tetrazol-5-yl)-phenyl]-2-oxo-5-oxazolidinyl]methyl]acetamide;
*N*-[[(5S)-3-[3-fluoro-4-(1-methoxycarbonylmethyl-1*H*-tetrazol-5-yl)-phenyl]-2-oxo-5-oxazolidinyl]methyl]acetamide;
*N*-[[(5S)-3-[3-fluoro-4-(2-propargyl-2*H*-tetrazol-5-yl)-phenyl]-2-oxo-5-oxazol id inyl] methyl]acetam ide;
*N*-[[(5S)-3-[3-fluoro-4-(1-propargyl-1*H*-tetrazol-5-yl)-phenyl]-2-oxo-5-oxazol id inyl] methyl]acetam ide;
*N*-[[(5S)-3-[3-fluoro-4-(2-carboxymethyl-2*H*-tetrazol-5-yl)-phenyl]-2-oxo-5-oxazolidinyl]methyl]acetamide; and
*N*-[[(5S)-3-[3-fluoro-4-(1-carboxymethyl-1*H*-tetrazol-5-yl)-phenyl]-2-oxo-5-oxazolidinyl]methyl]acetamide; or
   pharmaceutically acceptable salts thereof.

Compounds of formula (I) can be prepared by a process comprising the following steps:
a) cycling a compound of formula (II) 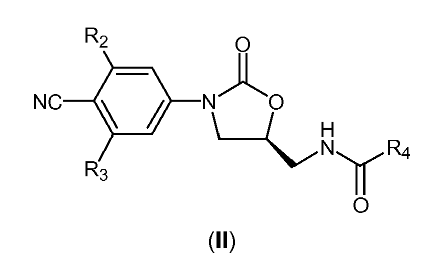 wherein R₂, R₃ and R₄ are as defined above, with Bu₃SnN₃ to afford a compound of formula (III) 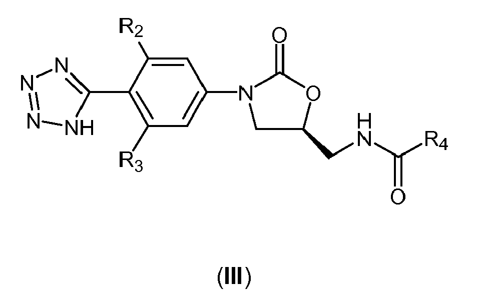 wherein R₂, R₃ and R₄ are as defined above;
b) when necessary, alkylating said compound of formula (III) with an alkylating compound of formula L-(R'₅)n, wherein L is a monovalent or divalent leaving group and R'₅ is a radical selected from the group consisting of a linear or branched alkyl (C₁-C₆) optionally substituted by OH, OR₆, NO₂, CF₃, CN, COOH, COOR₇, COR₈, CONH₂, CONHR₉, and CONR₁₀R₁₁, wherein R₆, R₇, R₈, R₉ and R₁₀ and R₁₁ are as defined above; and n is 1 or 2; and
c) separating the resulting regioisomers if necessary.

Convenient monovalent leaving groups are selected from halogens, sulfonates and carboxylates. Thus, in another embodiment, L is a representative monovalent leaving group selected from the group consisting of halogens such as chlorine, bromine, and iodine, sulfonates such as mesylate, besylate, tosylate, brosylate, and nosylate, and the like, and carboxylates such as acetate, and triflate, and the like, then being n=1.

In another embodiment, L is the divalent leaving group sulfate, then being n=2.

The present invention also provides a process for the preparation of a compound of formula (**IV**) 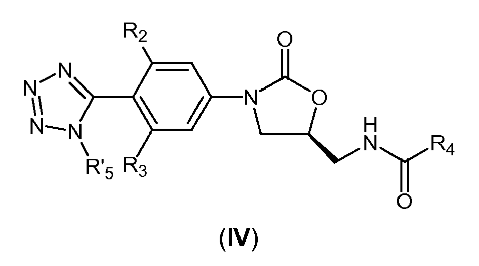 wherein R₂, R₃, R₄ and R'₅ are as defined above, by coupling a compound of formula (**V**) 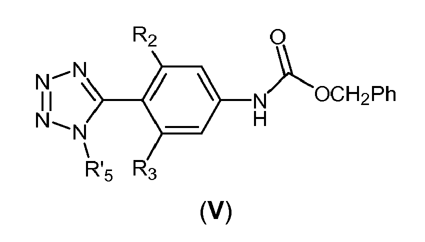 wherein R₂, R₃ and R'₅ are as defined above,
with a compound of formula (**VI**) 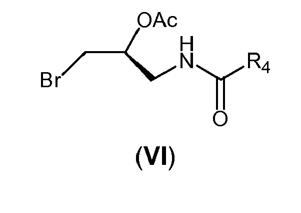 wherein R₄ is as defined above.

The term "pharmaceutically acceptable salts" used herein encompasses any salt formed from organic and inorganic acids or from organic or inorganic bases.

Exemplary organic and inorganic acids forming salts include hydrobromic, hydrochloric, phosphoric, nitric, sulfuric, acetic, adipic, aspartic, benzenesulfonic, benzoic, citric, ethanesulfonic, formic, fumaric, glutamic, lactic, maleic, malic, malonic, mandelic, methanesulfonic, 1,5-naphthalendisulfonic, oxalic, pivalic, propionic, p-toluenesulfonic, succinic, and tartaric, and the like.

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamines, t-butyl amines, and salts with amino acids such as arginine, lysine, and the like.

According to precedent embodiments, the compounds of the present invention can be obtained through two experimental processes. The first approach affords the two possible regioisomers on the tetrazole moiety as isolated compounds from the same reaction mixture (Scheme 1). For instance, tetrazole derivative of Example 1 (**I**, R₁=(i), R₂=F, R₃=R₅=H, R₄=CH₃) can be obtained from the cyano derivative **1** (**II**, R₂=F, R₃=H, R₄=CH₃) already described elsewhere. Alkylation of tetrazole derivative **1** gives the 2- and 3- substituted tetrazole products which could be isolated by column chromatography. The structure of final compounds was assigned by 2D NMR experiments (COSY, HMQC).

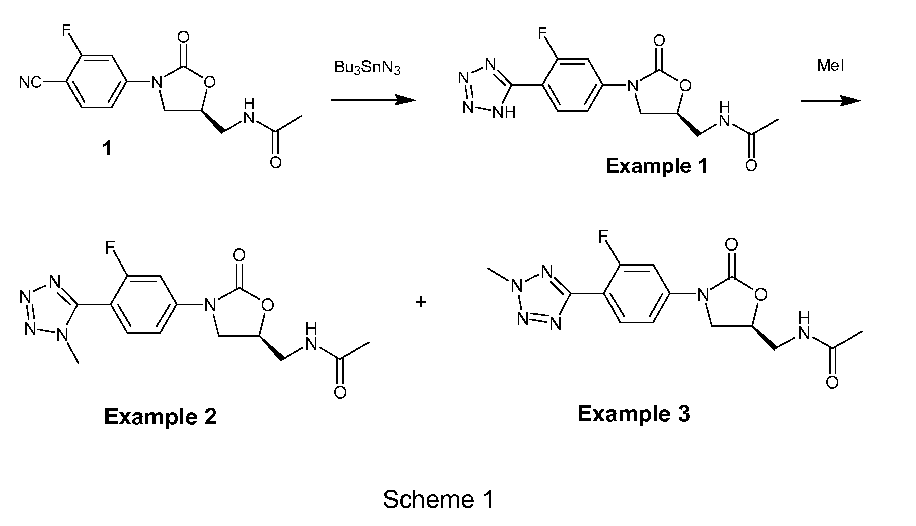

Moreover, the compound of Example 2 was also obtained by forming first the 2-Methyl-tetrazole moiety and after the oxazolidinone part (Scheme 2). In this reaction scheme only the 2-substituted tetrazole is obtained.

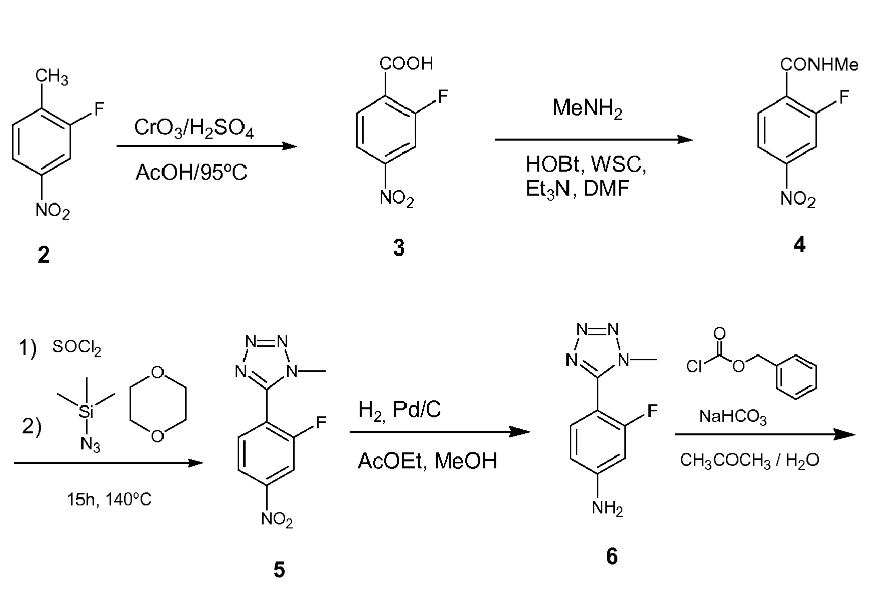

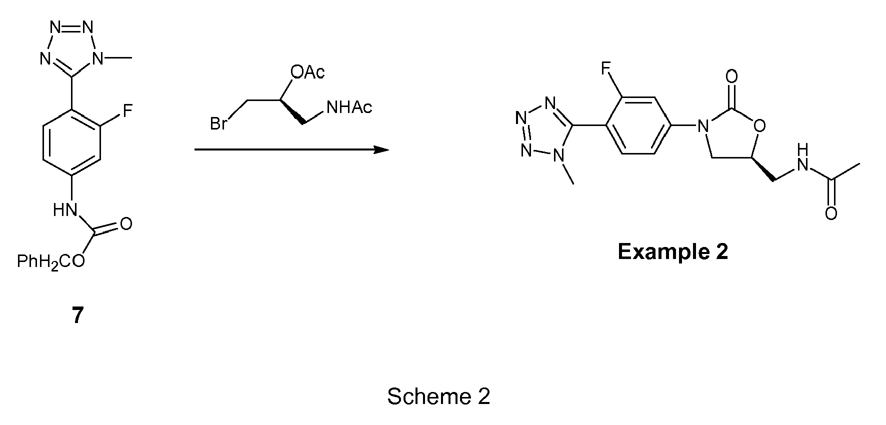

It is known that the substitution of phenyl-oxazolidinones with Carbon-linked azoles has resulted in the discovery of a new class of potent antibacterial agents (Hauck S. I. et al., Bioorg. Med. Chem. Lett. 17 (2007) 337-340). Unfortunately, an enhanced MAO activity over linezolid has been shown for these compounds. Surprisingly, the present invention provides a new subclass of phenyl-oxazolidinones with Carbon-linked azoles with not only a potent antibacterial activity but also a very low MAO inhibitory activity, which implies that potential drug-drug interactions are minimized or eliminated, and therefore, the side effects related to these interactions are avoided, too. Therefore, as it is illustrated below, the compounds of the invention show improved properties over other oxazolidinones with antibacterial activity known in the state of the art.

Thus, another aspect of the invention is to provide the use of a compound of formula (I) for the manufacture of a medicament for the treatment of bacterial infections in a human or animal. This aspect of the invention can also be formulated as compound as defined above for use as antibacterial compound.

The invention also relates to a method of treatment and/or prophylaxis of an animal or a human, suffering from or being susceptible to a bacterial infection, said method comprising the administration to said patient of a therapeutically effective amount of the compounds of the present invention, together with pharmaceutically acceptable excipients or carriers.

The compounds of the present invention can be normally formulated in accordance with standard pharmaceutical practice as a pharmaceutical composition.

The pharmaceutical compositions of this invention may be administered in standard manner for the disease condition that it is desired to treat, for example by oral, parenteral, inhalatory, rectal, transdermal or topical administration. For these purposes the compounds of this invention may be formulated by means known in the art in the form of, for example, tablets, capsules, syrups, aqueous or oily solutions or suspensions, emulsions, dispersible powders, inhalatory solutions, suppositories, ointments, creams, drops and sterile aqueous or oily solutions or suspensions for injection and the like. The pharmaceutical compositions may contain flavoring agents, sweeteners, etc. in suitable solid or liquid carriers or diluents, or in a suitable sterile media to form suspensions or solutions suitable for intravenous, subcutaneous or intramuscular injection. Such compositions typically contain from 1 to 40%, preferably 1 to 10% by weight of active compound, the remainder of the composition being pharmaceutically acceptable carriers, diluents, solvents and the like.

The compounds of formula (I) are administered in an amount of 0.1 to 100 mg/kg of body weight/day, preferably 1 to 50 mg/kg of body weight/day.

The compounds and compositions of the present invention are useful in the treatment of conditions such as nosocomial pneumoniae, community acquired pneumoniae, caused by methicillin-resistant *Staphylococcus aureus* (MRSA), including concurrent bacteremia, penicillin resistance and sensitive streptococcus pneumoniae, diabetic foot infections and skin and skin structure infections, and all other infections caused by bacteria sensitive to the compounds described in the invention. The compounds of the present invention are effective against a number of human or animal pathogens, clinical isolates, including vancomycin-resistant organisms and methicillin-resistant organisms. The pharmacological activity of the compounds of the present invention has been determined as shown in the

### examples.

Throughout the descrip!ion and claims the word "comprise" and variations of the word, such as "comprising", are not intended to exclude other technical features, additives, components, or steps. Additional objects, advantages and features of the invention will become apparent to those skilled in the art upon examination of the descrip!ion or may be learned by practice of the invention. The following examples are provided by way of illustration, and are not intended to be limiting of the present invention.

### EXAMPLES:

**Example 1:** *N*-[[(5S)-3-[3-fluoro-4-(1*H*-tetrazol-5-yl)-phenyl]-2-oxo-5-oxazolidinyl]methyl]acetamide

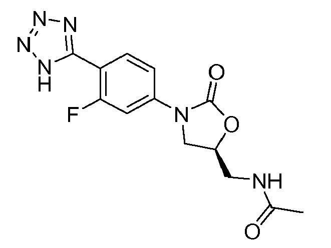

The *N*-[[(5S)-3-[3-fluoro-4-nitrile-phenyl]-2-oxo-5-oxazolidinyl]methyl] acetamide (290 mg), prepared as described in International Publication number WO 99/02525, is dissolved in toluene at room temperature. Tributyl tin azide (382 mg, 1.1 eq) is added and the resulting solution is stirred at reflux for 42 hours. 1.3 mL of NaOH 1N is added to the resulting heterogeneous crude mixture following of sonication. The heterogeneous aqueous layer is filtered and washed with toluene and dichloromethane.

The phases are separated and the aqueous layer is treated with a solution of HCl 1 N until pH=1 when a solid precipitates. The filter cake is washed with water and dried under vacuum at 40°C to afford 192 mg of the title compound.
HPLC (t, %): 0.56 min, 97%.
MS(ESI) m/z = 321(M+1)

**Example 2:** *N*-[[(5S)-3-[3-fluoro-4-(1-methyl-1*H*-tetrazol-5-yl)-phenyl]-2-oxo-5-oxazolidinyl]methyl]acetamide

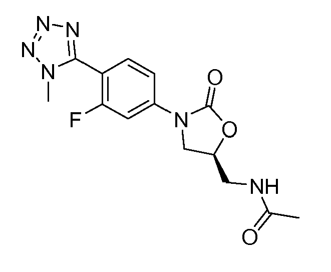

This compound can be prepared by two processes:

### Process A

Freshly calcined K2CO3 (43 mmol) was added to a solution of the N-[[(5S)-3-[3-fluoro-4-(1*H*-tetrazol-5-yl)-phenyl]-2-oxo-5-oxazolidinyl]methyl] acetamide (17.2 mmol) in 150 mL of dry dimethylacetamide. The mixture was stirred for 5 minutes and then methyl iodide (26 mmol) was added to the reaction mixture. The latter was heated at 80°C for three hours then cooled to room temperature, diluted with ethyl acetate and washed with water. The organic layer is washed with brine (three times, 100 mL) and the aqueous layers are extracted thoroughly with dichloromethane (three times, 100 mL). The combined organic layers are dried over MgSO₄ and concentrated under reduced pressure. The mixture of regioisomers was purified by column chromatography (silica gel, dichloromethane/MeOH 50:1) to give 0.85 gr of the title compound (Yield = 15%).
HPLC (t, %): 1.7 min, 98%.
MS(ESI) *m*/*z* = 335 (M+1).
¹H NMR (400 MHz, δ, ppm, DMSO):1.83 (3H, s), 3.44 (2H, t, J = 5.6 Hz), 3.81 (2H, m), 4.03 (3H, d, J = 1.2 Hz), 4.19 (1 H, t, J = 9.2 Hz), 4.75 (1 H, m), 7.56 (1 H, dd, J = 8.4, 2 Hz), 7.76 (2H, m), 8.26 (1 H, NH, t).

### Process B

### 2-Fluoro-4-nitro benzoic acid, 3

To a 250-mL round-bottomed flask fitted with magnetic stirrer, reflux condenser, thermometer and dropping funnel is slowly added 9.7 mL of concentrated sulfuric acid over a solution of 2-fluoro-4-nitrotoluene (5 g) in 61 mL of acetic acid. The mixture is heated to 100 °C when a water solution of CrO₃ is added during approximately 1 hour through the dropping funnel. The mixture is stirred for 1 hour more maintaining temperature below 95°C. The reaction mixture is cooled by use of an ice bath, dichloromethane is added and the resulting mixture is transferred to a separatory funnel. The aqueous layer is extracted twice with dichloromethane and the aqueous layer is separated and discarded. The organic layer is treated with saturated solution of Na₂CO₃, the resulting aqueous layer is acidified with concentrated HCl giving a pale yellow precipitate. The solid is filtered washed with cold water, and dried in a dessicator (50°C, 10 mm Hg, 24 h). The crude title compound (3.6 g, 60%) was used without further purification.
HPLC (t, %): 1.6 min, 100%.
MS(ESI) *m*/*z* = 184 (M-1).
¹H NMR (400 MHz, δ, ppm, DMSO): 8.10 (2H, m), 8.19 (1 H, d, J = 8.4 Hz).

### N-Methyl-2-Fluoro-4-nitro benzamide, 4

A solution of 1 g of 2-Fluoro-4-nitro benzoic acid, 0.93 gr of hydroxyl benzotriazole, 0.47 gr of methylamine hydrochloride, 0.9 mL of triethylamine and 1.35 g 1-ethyl-3-(3'-dimethylaminopropyl)carbodiimide hydrochloride (water soluble carbodiimide, WSC) in 30 mL of DMF was stirred at room temperature under argon for 24 hours. Complete conversion of the starting acid was observed by TLC and HPLC-MS. The crude mixture was washed with water, HCl 1 M, saturated solution of NaHC03, and brine. The combined organic layers were dried (MgSO4) and concentrated in vacuum to afford 0.92 g of title compound.
HPLC (t, %): 5.77 min, 100%.
MS(ESI) m/z = 199 (M+1).
¹H NMR (400 MHz, δ, ppm, DMSO): 2.79 (3H, d, J = 4.8 MHz), 7.83 (1H, m), 8.12 (1 H. m), 8.19 (1 H, dd, J = 10, 2 Hz), 8.59 (1 H, NH).

### 5-(2-Fluoro-4-nitro-phenyl)-1-methyl-1H-tetrazole, 5

A solution of 1 g *N*-Methyl-2-Fluoro-4-nitro benzamide in 5 mL of thionylchloride was heated under microwave at 100 °C for 30 minutes. The reaction mixture was concentrated under vacuum. To this residue, 0.99 mL of trimethylsilyl azide and 15 mL of dioxane were added and heated to 140°C under microwave for 15 hours. The crude mixture is concentrated under vacuum and purified by column chromatography (Merck silica gel, dichloromethane/AcOEt increasing polarity) to afford 500 mg of title compound (Yield= 45%).
HPLC (t, %): 6.49 min, 95%.
MS(ESI) *m*/*z* = 224 (M+1).
¹H NMR (400 MHz, δ, ppm, DMSO): 4.08 (3H, s), 8.08 (1H, t, J = 8 Hz), 8.30 (1 H, dd, J = 8.4, 1.2 Hz), 8.44 (1 H, dd, J = 4.8, 1 Hz).

### 3-Fluoro-4-(1-methyl-1H-tetrazole-5yl)-phenylamine, 6

A solution of 480 mg of 5-(2-Fluoro-4-nitro-phenyl)-1-methyl-1*H*-tetrazole and 50 mg of Pd/C (10%) in 20 mL of Methanol/Ethyl Acetate (1:1) was stirred under hydrogen atmosphere for two hours when completed reaction was detected by TLC. The palladium on carbon was filtered off and the filtrate was concentrated under vacuum to afford 400 mg of title compound.
HPLC (t, %): 5.51 min, 100%.
MS(ESI) m/z = 194 (M+1).
¹H NMR (400 MHz, δ, ppm, DMSO): 3.96 (3H, d, J = 1.6 Hz), 6.16 (1H, s), 6.52(2H, m), 7.29 (1 H, t, J = 8.4 Hz).

### 3-Fluoro-4-(1-methyl-1H-tetrazole-5yl)-phenyl carbamic acid benzyl ester, 7

0.68 g of Sodium hydrogencarbonate and 0.72 mL of benzyloxycarbonyl chloride were added successively at 0°C to an acetone solution (10 mL) of 3-Fluoro-4-(1-methyl-1*H*-tetrazole-5yl)-phenylamine. The mixture was stirred at room temperature for 14 hours until completed reaction was detected by TLC. Water was added to the reaction solution and the separated organic layer was washed with water, brine and dried over anhydrous magnesium sulphate. The solvent was evaporated and the residue was purified by silica gel column chromatography (dichloromethane, Ethyl Acetate in increasing polarity) to afford 498 mg of title compound.
HPLC (t, %): 8.2 min, 87%.
MS(ESI) m/z = 328 (M+1).
¹H NMR (400 MHz, δ, ppm, DMSO): 4.01 (3H, d, J = 1.6 Hz), 5.19 (2H, s), 7.4 (6H, m), 7.65 (2H, m), 10.42 (1 H, NH).

### N-[[(5S)-3-[3-fluoro-4-(1-methyl-1H-tetrazol-5-yl)-phenyl]-2-oxo-5-oxazolidinyl]methyl]acetamide

To a solution of 50 mg of 3-Fluoro-4-(1-methyl-1*H*-tetrazole-5-yl)-phenyl carbamic acid benzyl ester in 100 L of DMF was added 458 L of a solution 1 M of lithium *tert*-butoxide in THF and stirred at room temperature for 30 minutes. 12 I of methanol and a solution of 72.7 mg of (S)-N-(3-bromo-2-acetoxypropyl)acetamide were added and allowed to stand at room temperature for two days at which point HPLC showed a 30% conversion. The same amount of lithium *tert*-butoxide in THF, methanol and (S)-N-(3-bromo-2-acetoxypropyl)acetamide were added and the reaction mixture stirred at room temperature for two days more. Saturated aqueous ammonium chloride was added to the reaction solution and the separated organic layer was washed with water, brine and dried over anhydrous magnesium sulphate. The solvent was evaporated and the residue was purified by silica gel column chromatography (dichloromethane, Ethyl Acetate in increasing polarity) to afford the title compound.

### Example 3: N-[[(5S)-3-[3-fluoro-4-(2-methyl-2H-tetrazol-5-yl)-phenyl]-2-oxo-5-oxazolidinyl]methyl]acetamide

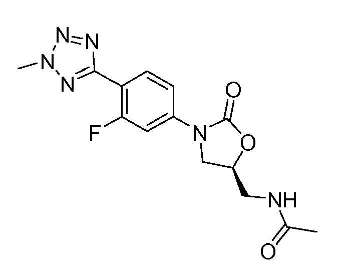

It was obtained concomitantly with compound of Example 2, which after column chromatography afforded 1.85 gr of title compound (Yield = 32%).
HPLC (t, %): 1.9 min, 99%.
MS(ESI) m/z = 335 (M+1).
¹H NMR (400 MHz, δ,, ppm, DMSO): 1.83 (3H, s), 3.43 (2H, t, J = 5.2 Hz), 3.79 (1 H, m), 4.18 (1 H, t, J = 8.8 Hz), 4.18 (1 H, t, J = Hz), 4.43 (3H, s), 4.78 (1 H, m), 7.52 (1 H, dd, J = 8.8, 2.4 Hz), 7.69 (1 H, dd, J = 13.2, 2.4 Hz), 8.07 (1 H, t, J = 8.5 Hz), 8.25 (1 H, NH, t).

### Example 4: N-[[(5S)-3-[3-fluoro-4-(2-cyanomethyl-2H-tetrazol-5-yl)-phenyl]-2-oxo-5-oxazolidinyl]methyl]acetamide

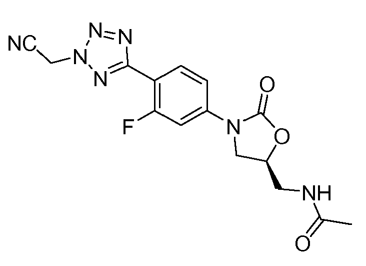

Freshly calcined K₂CO₃ (2.5 equivalents) was added to a solution of the N-[[(5S)-3-[3-fluoro-4-(1*H*-tetrazol-5-yl)-phenyl]-2-oxo-5-oxazolidinyl]methyl]acetamide (0.84 mmol) in 4 mL of dry dimethylacetamide. The mixture was stirred for 5 minutes and then chloroacetonitrile (1.5 equivalents) was added to the reaction mixture. The latter was heated at 80°C under microwave conditions for one hour then cooled to room temperature, diluted with ethyl acetate and washed with water. The organic layer is washed with brine and the aqueous layers are extracted thoroughly with dichloromethane. The combined organic layers are dried over MgSO₄ and concentrated under reduced pressure. The mixture of regioisomers was purified by column chromatography (silica gel, dichloromethane/MeOH increasing polarity) to give 89 mg of the title compound as a major regioisomer.
HPLC (t, %): 2.0 min, 98%.
MS(ESI) m/z = 360 (M+1).

### Example 5: N-[[(5S)-3-[3-fluoro-4-(1-cyanomethyl-1H-tetrazol-5-yl)-phenyl]-2-oxo-5-oxazolidinyl]methyl]acetamide

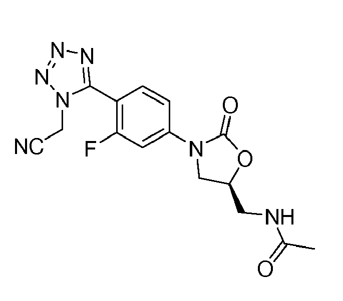

It was obtained concomitantly with compound of Example 4, which after column chromatography afforded 30 mg of title compound.
HPLC (t, %): 1.8 min, 93%.
MS(ESI) m/z = 360 (M+1).

### Example 6: N-[[(5S)-3-[3-fluoro-4-(2-acetoacetyl-2H-tetrazol-5-yl)-phenyl]-2-oxo-5-oxazolidinyl]methyl]acetamide

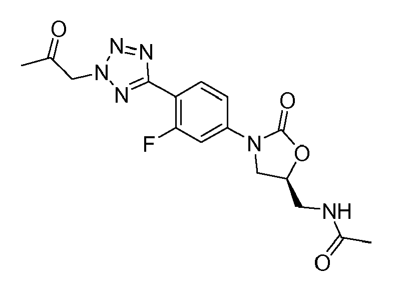

It was prepared following the same process as in Example 4, obtaining 5 mg of major regioisomer.
HPLC (t, %): 1.18 min, 99%.
MS(ESI) m/z = 365 (M+1).

### Example 7: N-[[(5S)-3-[3-fluoro-4-(2-hydroxyethyl-2H-tetrazol-5-yl)-phenyl]-2-oxo-5-oxazolidinyl]methyl]acetamide

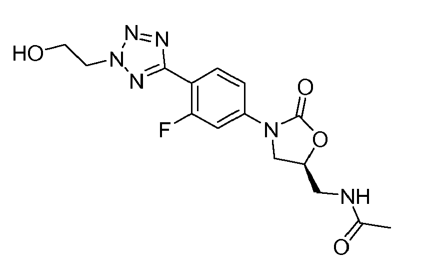

It was prepared following the same process as in Example 4, obtaining 25 mg of title compound.
HPLC (t, %): 1.12 min, 99%.
MS(ESI) m/z = 365 (M+1)

### Example 8: N-[[(5S)-3-[3-fluoro-4-(1-hydroxyethyl-1H-tetrazol-5-yl)-phenyl]-2-oxo-5-oxazolidinyl]methyl]acetamide

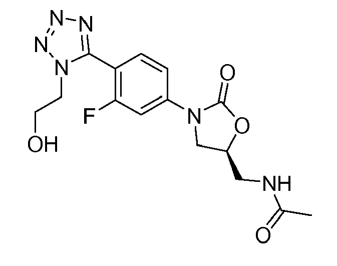

It was obtained concomitantly with compound of Example 7, which after column chromatography afforded 5 mg of title compound.
HPLC (t, %): 1.0 min, 93%.

MS(ESI) m/z = 365 (M+1)

### Example 9: N-[[(5S)-3-[3-fluoro-4-(2-methoxyethyl-2H-tetrazol-5-yl)-phenyl]-2-oxo-5-oxazolidinyl]methyl]acetamide

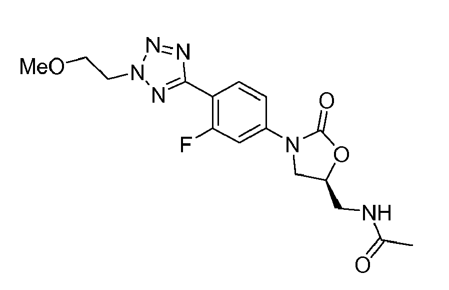

It was prepared following the same process as in Example 4, obtaining 13 mg of title compound.
HPLC (t, %): 1.5 min, 99%.
MS(ESI) m/z = 379 (M+1)

### Example 10: N-[[(5S)-3-[3-fluoro-4-(1-methoxyethyl-1H-tetrazol-5-yl)-phenyl]-2-oxo-5-oxazolidinyl]methyl]acetamide

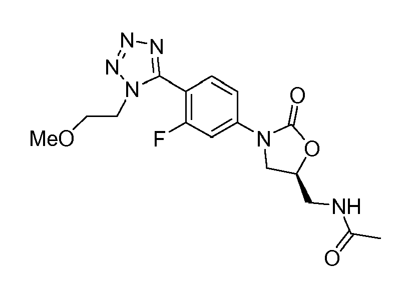

It was obtained concomitantly with compound of Example 9, which after column chromatography afforded 1 mg of title compound.
HPLC (t, %): 1.3 min, 93%.
MS(ESI) m/z = 379 (M+1)

### Example 11: N-[[(5S)-3-[3-fluoro-4-(2-methoxycarbonylmethyl-2H-tetrazol-5-yl)-phenyl]-2-oxo-5-oxazolidinyl]methyl]acetamide

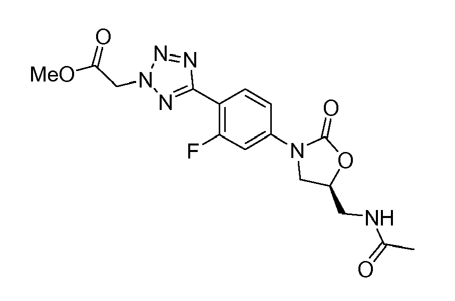

It was prepared following the same process as in Example 4, obtaining 17 mg of title compound.
HPLC (t, %): 1.55 min, 99%.
MS(ESI) m/z = 393 (M+1)

### Example 12: N-[[(5S)-3-[3-fluoro-4-(1-methoxycarbonylmethyl-1H-tetrazol-5-yl)-phenyl]-2-oxo-5-oxazolidinyl]methyl]acetamide

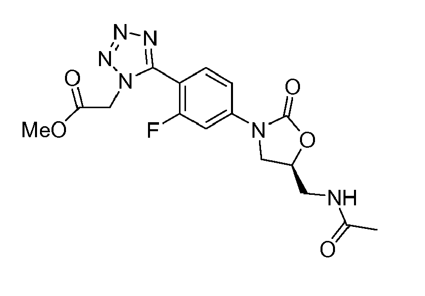

It was obtained concomitantly with compound of Example 11, which after column chromatography afforded 4 mg of title compound.
HPLC (t, %): 1.35 min, 93%.
MS(ESI) m/z = 393 (M+1)

### Example 13: N-[[(5S)-3-[3-fluoro-4-(2-propargyl-2H-tetrazol-5-yl)-phenyl]-2-oxo-5-oxazolidinyl]methyl]acetamide

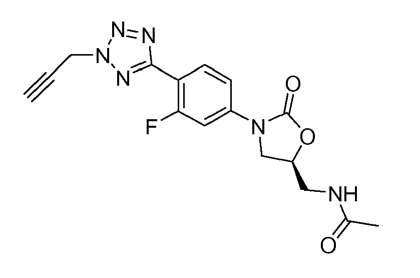

It was prepared following the same process as in Example 4, obtaining 37 mg of title compound.
HPLC (t, %): 1.9 min, 99%.
MS(ESI) m/z = 359 (M+1)

### Example 14: N-[[(5S)-3-[3-fluoro-4-(1-propargyl-1H-tetrazol-5-yl)-phenyl]-2-oxo-5-oxazolidinyl]methyl]acetamide

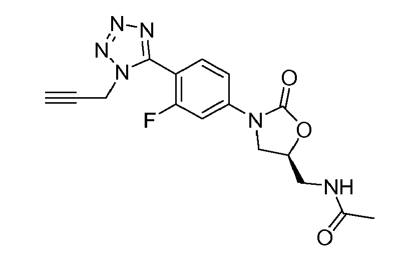

It was obtained concomitantly with compound of Example 13, which after column chromatography afforded 8 mg of title compound.
HPLC (t, %): 1.67 min, 99%.
MS(ESI) m/z = 359 (M+1)

### Example 15: N-[[(5S)-3-[3-fluoro-4-(2-carboxymethyl-2H-tetrazol-5-yl)-phenyl]-2-oxo-5-oxazolidinyl]methyl]acetamide

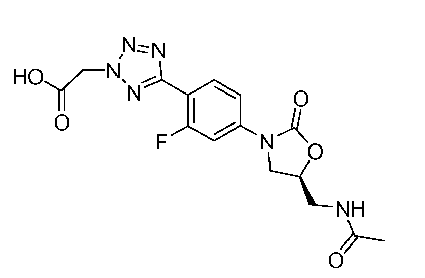

It was prepared following the same process as in Example 4, obtaining 2 mg of title compound.
HPLC (t, %): 1.18 min, 98%.
MS(ESI) m/z = 379 (M+1)

### Example 16: N-[[(5S)-3-[3-fluoro-4-(1-carboxymethyl-1H-tetrazol-5-yl)-phenyl]-2-oxo-5-oxazolidinyl]methyl]acetamide

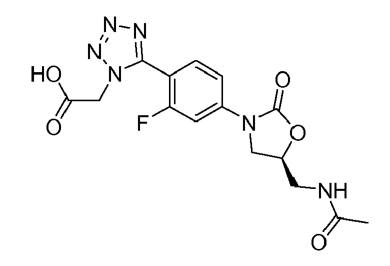

It was obtained concomitantly with compound of Example 15, which after column chromatography afforded 8 mg of title compound.
HPLC (t, %): 0.94 min, 97%.
MS(ESI) m/z = 379 (M+1)

### Example 17: Antibacterial activity

MICs were determined by using a standard micro dilution method according to The National Committee for Clinical Laboratory Standards (NCCLS), 5^{th} Approved standard M7-A5, 2001, Wayne, PA, USA. All compounds were tested against Gram-positive and Gram-negative bacteria showing relevant different susceptibility and resistance specifications. The used microorganisms were selected from laboratory reference bacteria and from clinical isolates.The tested concentrations were double dilutions from 0.06 µg/mL to 128 µg/mL in 96-well micro titter plates.
The microorganisms used in the study were:
Aerobic Gram-positive bacteria, consisting of *Staphylococcus aureus, Staphylococcus epidermidis,* and *Streptococcus pneumoniae.*

MICs were determined in the *Brucella* Blood medium supplemented for the anaerobic strains, and in the Mueller-Hinton culture medium (cation-adjusted) for the aerobic bacteria.

The tested compounds were dissolved in dimethylsulfoxide, and were diluted as far as 2560 µg/mL with the different media according to the specific requirements for each group of strains. The 96-well sealed micro titter plates containing bacteria were incubated in different laboratory conditions depending on the nature of the micro organism. Thus, the aerobic bacteria were incubated during 16-24 h at 35°C and the so-called fastidious bacteria, such as M. *catarrhalis* and *S. pneumoniae,* during 20-24h at 35°C in a microaerobiotic atmosphere containing 5% CO₂ (Anaerocult C, MERCK).
The results of these tests are given in Table 1.

**Table 1. In vitro activities against bacteria**

| *Geometric Mean MIC µg*/*mL* | | | |
|---|---|---|---|
| *Compound* | *S. Aureus MR* | *S. epidermidis* | *S. pneumoniae* |
| Example 2 | 2 | 2 | 2 |
| Example 8 | 2 | 1 | 2 |
| Linezolid | 1 | 1 | 1 |

### Example 18: In Vitro MAO-A and MAO-B enzymatic activity

MAO-A and MAO-B enzymatic activities were measured using membranes obtained from SF9 cells expressing either human MAO-A or human MAO-B (Gentest, BD, USA). Assays were done in blank 96-well micro titter plates using kynuramine as substrate and measuring the formation of 4-hydroxyquinoline by fluorescence at 340 nm/465 nm. Briefly, membranes with MAO-A (0.006 mg/mL protein) and MAO-B (0.015 mg/mL protein) were incubated with kynuramine, 30 µM), at 37° for 40 min in the presence of the compound in a final volume of 200 µL. Reactions were stopped by adding NaOH 2N and the reaction product, 4-hydroxyquinoline, was determined by fluorometry using a Tecan Ultra reader.

A low Kᵢ value indicates that the tested inhibitor possesses a tight binding ability to MAO enzyme, thus, it is a strong MAO inhibitor.

MAO-A and MAO-B enzymatic activities are shown in Table 2.

**Table 2. Inhibitory activity on human MAO**

| *Compound* | *MAO-A Ki uM* | *MAO-B Ki uM* |
|---|---|---|
| Example 2 | 74.2 | 14.2. |
| Example 6 | 37.1 | 10.1 |
| Example 7 | 28.8 | 8.10 |
| Linezolid | 6.96 | 0.48 |

### Example 19: Pharmaceutical compositions

The following illustrate representative pharmaceutical compositions containing a compound of formula (I) or a pharmaceutically acceptable salt thereof for antimicrobial use in humans or animals:

| Tablet 1 | mg/tablet |
|---|---|
| Active ingredient | 100 |
| Lactose | 179 |
| Croscarmellose sodium | 12 |
| Polyvinylpyrrolidone | 6 |
| Magnesium stearate | 3 |

| Tablet 2 | mg/tablet |
|---|---|
| Active ingredient | 50 |
| Lactose | 229 |
| Croscarmellose sodium | 12 |
| Polyvinylpyrrolidone | 6 |
| Magnesium stearate | 3 |

| Tablet 3 | mg/tablet |
|---|---|
| Active ingredient | 1 |
| Lactose | 92 |
| Croscarmellose sodium | 4 |
| Polyvinylpyrrolidone | 2 |
| Magnesium stearate | 1 |

| Capsule | mg/capsule |
|---|---|
| Active ingredient | 10 |
| Lactose | 389 |
| Croscarmellose sodium | 100 |
| Magnesium stearate | 1 |

| Injection | 50 mg/mL |
|---|---|
| Active ingredient | 5.0% w/v |
| Isotonic aqueous solution | to 100% |

Buffers, pharmaceutically acceptable co-solvents such as polyethylene glycol, polypropylene glycol, glycerol or ethanol or complexing agents, may be used to aid formulation.

The above formulations may be prepared by well-known conventional processs in the pharmaceutical art. The tablets 1-3 may be enteric coated by conventional means, for example to provide a coating of cellulose acetate phthalate.

## Claims

1. A compound of formula (I): 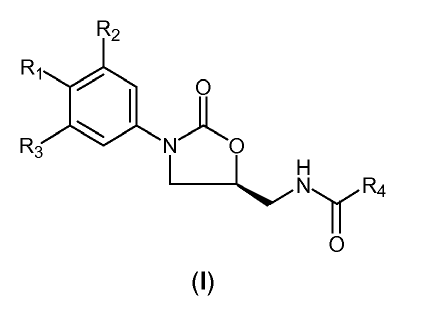 or a pharmaceutically acceptable salt thereof, wherein:
R₁ is a radical of formula (i) or formula (ii): 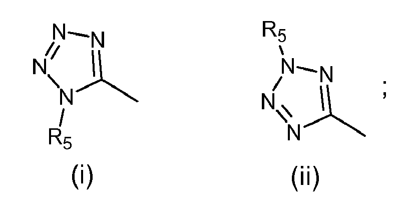
R₂ and R₃ are radicals identical or different and are independently selected from hydrogen and fluorine;
R₄ is a linear or branched alkyl (C₁-C₆) radical optionally substituted by 1 to 3 fluorine atoms;
R₅ is a radical selected from the group consisting of hydrogen; linear or branched alkyl (C₁-C₆) optionally substituted by OH, OR₆, NO₂, CF₃, CN, COOH, COOR₇, COR₈, CONH₂, CONHR₉, and CONR₁₀R₁₁; and linear or branched alkynyl (C₁-C₆);
R₆ is a linear or branched alkyl (C₁-C₆) radical;
R₇ is a linear or branched alkyl (C₁-C₆) radical;
R₈ is a linear or branched alkyl (C₁-C₆) radical;
R₉ is a linear or branched alkyl (C₁-C₆) radical; and
R₁₀ and R₁₁ are linear or branched alkyl (C₁-C₆) radicals which are identical or different or taken together can form with the nitrogen atom to which they are attached a pyrrolidine or piperidine ring.

2. The compound of claim 1, wherein at least one of R₂ and R₃ is fluorine.

3. The compound of claim 1, wherein R₄ is a linear or branched alkyl (C₁-C₆) radical.

4. The compound of claim 1, wherein R₅ is hydrogen; a linear or branched alkyl (C₁-C₆) optionally substituted by OH, OR₆, CN, COOH, COOR₇, and COR₈; or a linear or branched alkynyl (C₁-C₆).

5. The compound of claim1, which is selected from the group consisting of:
*N*-[[(5S)-3-[3-fluoro-4-(1*H*-tetrazol-5-yl)-phenyl]-2-oxo-5-oxazol id inyl] methyl]acetam ide;
*N*-[[(5S)-3-[3-fluoro-4-(1-methyl-1*H*-tetrazol-5-yl)-phenyl]-2-oxo-5-oxazol id inyl] methyl]acetam ide;
N-[[(5S)-3-[3-fluoro-4-(2-methyl-2H-tetrazol-5-yl)-phenyl]-2-oxo-5-oxazol id inyl] methyl]acetam ide;
*N*-[[(5S)-3-[3-fluoro-4-(2-cyanomethyl-2*H*-tetrazol-5-yl)-phenyl]-2-oxo-5-oxazol id inyl] methyl]acetam ide;
*N*-[[(5S)-3-[3-fluoro-4-(1-cyanomethyl-1*H*-tetrazol-5-yl)-phenyl]-2-oxo-5-oxazolidinyl]methyl]acetamide;
*N*-[[(5S)-3-[3-fluoro-4-(2-acetoacetyl-2*H*-tetrazol-5-yl)-phenyl]-2-oxo-5-oxazol id inyl] methyl]acetam ide;
*N*-[[(5S)-3-[3-fluoro-4-(2-hydroxyethyl-2*H*-tetrazol-5-yl)-phenyl]-2-oxo-5-oxazol id inyl] methyl]acetam ide;
*N*-[[(5S)-3-[3-fluoro-4-(1-hydroxyethyl-1*H*-tetrazol-5-yl)-phenyl]-2-oxo-5-oxazol id inyl] methyl]acetam ide;
*N*-[[(5S)-3-[3-fluoro-4-(2-methoxyethyl-2*H*-tetrazol-5-yl)-phenyl]-2-oxo-5-oxazol id inyl] methyl]acetam ide;
*N*-[[(5S)-3-[3-fluoro-4-(1-methoxyethyl-1*H*-tetrazol-5-yl)-phenyl]-2-oxo-5-oxazol id inyl] methyl]acetam ide;
*N*-[[(5S)-3-[3-fluoro-4-(2-methoxycarbonylmethyl-2*H*-tetrazol-5-yl)-phenyl]-2-oxo-5-oxazolidinyl]methyl]acetamide;
*N*-[[(5S)-3-[3-fluoro-4-(1-methoxycarbonylmethyl-1*H*-tetrazol-5-yl)-phenyl]-2-oxo-5-oxazolidinyl]methyl]acetamide;
*N*-[[(5S)-3-[3-fluoro-4-(2-propargyl-2*H*-tetrazol-5-yl)-phenyl]-2-oxo-5-oxazol id inyl] methyl]acetam ide;
*N*-[[(5S)-3-[3-fluoro-4-(1-propargyl-1*H*-tetrazol-5-yl)-phenyl]-2-oxo-5-oxazol id inyl] methyl]acetam ide;
*N*-[[(5S)-3-[3-fluoro-4-(2-carboxymethyl-2*H*-tetrazol-5-yl)-phenyl]-2-oxo-5-oxazolidinyl]methyl]acetamide; and
*N*-[[(5S)-3-[3-fluoro-4-(1-carboxymethyl-1*H*-tetrazol-5-yl)-phenyl]-2-oxo-5-oxazolidinyl]methyl]acetamide; or
pharmaceutically acceptable salts thereof.

6. A process for the preparation of a compound of claim 1, comprising the following steps:
a) cycling a compound of formula (II), 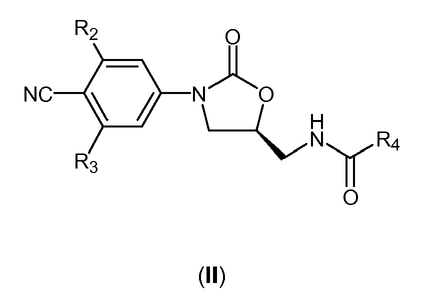 wherein R₂, R₃ and R₄ are as defined above, with Bu₃SnN₃ to afford a compound of formula (III) 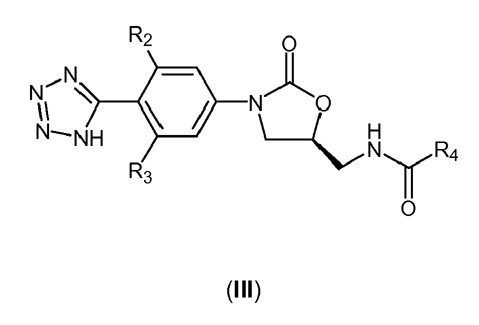 wherein R₂, R₃ and R₄ are as defined above;
b) when necessary, alkylating said compound of formula (III) with an alkylating compound of formula L-(R'₅)n, wherein L is a monovalent or divalent leaving group and R'₅ is a radical selected from the group consisting of a linear or branched alkyl (C₁-C₆) optionally substituted by OH, OR₆, NO₂, CF₃, CN, COOH, COOR₇, COR₈, CONH₂, CONHR₉, and CONR₁₀R₁₁, wherein R₆, R₇, R₈, R₉ and R₁₀ and R₁₁ are as defined above; and n is 1 or 2; and
c) separating the resulting regioisomers if necessary.

7. A process according to claim 6, wherein L is a monovalent leaving group selected from the group consisting of chlorine, bromine, iodine, mesylate, besylate, tosylate, brosylate, nosylate, acetate, and triflate, then being n=1.

8. A process according to claim 6, wherein L is the divalent leaving group sulfate, then being n=2.

9. A process for the preparation of a compound of formula (IV) 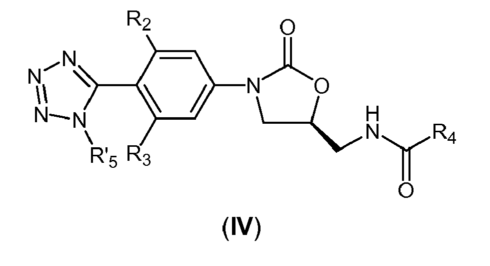 wherein R₂, R₃, R₄ and R'₅ are as defined above, by coupling a compound of formula (V) 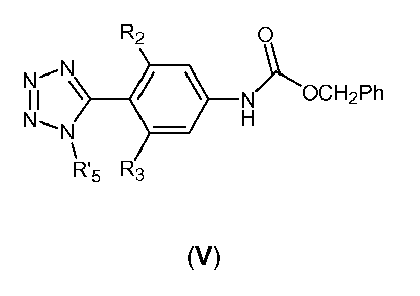 wherein R₂, R₃ and R'₅ are as defined above,
with a compound of formula (VI) 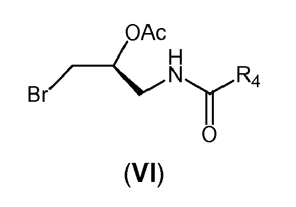 wherein R₄ is as defined above.

10. A pharmaceutical composition comprising at least one compound of claim 1, together with appropriate amounts of pharmaceutical excipients or carriers.

11. Use of a compound of claim 1, for the manufacture of a medicament for the treatment of bacterial infections in a human or animal.

12. Compound of formula (I) as defined in claim 1 for use as a medicament.

13. Compound of formula (I) as defined in claim 1, for use as antibacterial in a human or animal.
